(19) Europäisches Patentamt / European Patent Office / Office européen des brevets

(11) **EP 2 852 331 B1**

(12) **EUROPEAN PATENT SPECIFICATION**

(45) Date of publication and mention
of the grant of the patent:
**04.04.2018 Bulletin 2018/14**

(21) Application number: **13727443.7**

(22) Date of filing: **14.05.2013**

(51) Int Cl.:
*A61B 17/04* (2006.01)          *A61B 17/84* (2006.01)
*A61B 17/82* (2006.01)          *A61B 17/88* (2006.01)
*A61B 17/68* (2006.01)

(86) International application number:
**PCT/US2013/040989**

(87) International publication number:
**WO 2013/173365 (21.11.2013 Gazette 2013/47)**

(54) **HIGH TENSION SUTURE ANCHOR**

HOCHSPANNUNGSNAHTANKER

ANCRE DE SUTURE À HAUTE TENSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2012   US 201261741792 P
01.03.2013   US 201313783061**

(43) Date of publication of application:
**01.04.2015   Bulletin 2015/14**

(73) Proprietor: **Poly-4 Group, LP
Austin, TX 78746 (US)**

(72) Inventor: **MATTCHEN, Terry
Scottsdale, AZ 85258 (US)**

(74) Representative: **Leach, Sean Adam et al
Mathys & Squire LLP
The Shard
32 London Bridge Street
London SE1 9SG (GB)**

(56) References cited:
**EP-A2- 0 591 991          EP-A2- 1 016 377
EP-A2- 1 844 715          WO-A1-2010/014119
WO-A2-2005/124187          US-A- 5 258 015
US-A- 6 086 608          US-A1- 2008 004 624**

## Description

## BACKGROUND

## Field of the Invention

This invention relates to devices for retaining surgical cables under high tension, and tools and methods for implanting or otherwise installing such devices.

## Description of the Related Art

Present day polymer based surgical cables seem to defy all attempts to engage and lock the under high tension, thereby dramatically curtailing their range of practical use. Cable locks such as described in US 7,625,373 attempt to solve the just such a problem. US 7,625,373 is a typical example of the use of a wedge as a simple machine for securement.

Material properties of polymer fibers tend to complicate the situation. Surfaces tend to be slippery and materials have hysteresis. In some circumstances the materials tend to deform and flow (as implied by the common term "plastic"). Conventional knots are inadequate; clasps and fasteners slip under high tension. Attempts to counteract slippage by application of increased pressure are likely to result in cutting or fraying of the cable. Despite their usefulness, widespread acceptance of polymer cables depends in part on the availability of an efficient, economical, convenient, and reliable means of clamping and retaining under moderate to high tension.

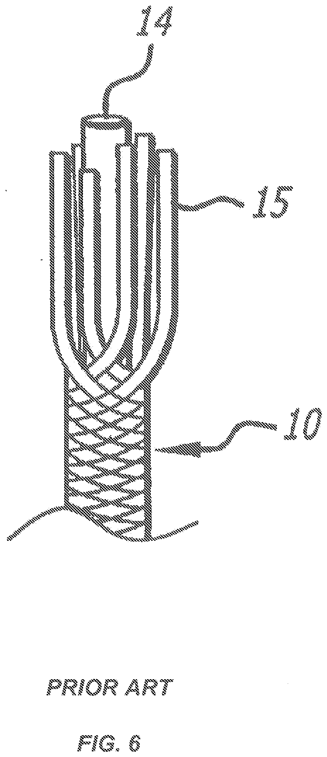

Nevertheless, as disclosed in US 6,589,246, certain polymer cables have shown promise for surgical use. The cable (10) of the '246 patent, shown in Figure 6, is a composite. The nylon or other polymeric material core (14) comprises about 75% of the diameter of the finished cable; the high strength UHMW polyethylene fibers (15) braided onto the core comprise the remaining 25%.

Examples of difficulties that may be unique to or more-pronounced while gripping such a braided polymer cable over the conventional steel cable are the following:

- The polymer cable stretches under high tension. As tension is applied, the diameter of the nylon or other polymeric material core may shrink by approximately 12%. Thus, the gripping device must be flexible enough to collapse correspondingly, all the while maintaining a firm grip on the cable of sufficient strength to counter the tension applied to the cable. By contrast, prior art steel cables do not shrink to such a pronounced degree (often not even to a perceptible degree) under tension, thereby generally obviating the need for a flexibly collapsing gripping mechanism.
- The fibers of the braided jacket do not stretch along with the core. Because such fibers are extremely delicate, (i.e., each fiber having a diameter measuring only a few ten-thousandth's of an inch), they are susceptible to breaking if not gripped gently and/or uniformly about the circumference of the cable. By contrast, prior art steel cables generally have a more-robust metallic surface and are therefore not sensitive or much-less sensitive to asymmetric gripping.
- Finally, the polyethylene fibers of the jacket are generally quite slippery, and so must be held gently, uniformly, and yet still quite firmly.

US 5 258 015 A discloses the features of the preamble of claim 1 and describes a locking cap for mounting on the smooth flexible filament of a surgical fastener wherein the locking cap is of a thin flat washer-like shape with gripping elements for engaging the filament and locking the cap in position relative to the filament.

US 2008/004624 A1 describes an anchoring fastener that is adapted for attaching an elastic cable to a substrate, and that is suitable for use in stabilizing and fixating a bony fracture with an elastic cable having known diameter and elastic properties.

## SUMMARY

The invention is defined in the appended claims. In essence, the force F required to retain a cable such as the one disclosed in the '246 Patent must be applied to the cable in such a way that the cable is not damaged, yet be of sufficiently significant magnitude to maintain a grip on the cable even when the cable is under high tension as the healing process evolves.

One way to accomplish this goal is to apply the force over a large surface area such that the force is distributed and the resulting pressure is reduced, i.e.

$$F = \int P \, da$$

where P is the applied pressure and the integral is taken over the contacting surface area of the cable. Although the pressure indeed will vary to some degree from point to point, it is desirable to maintain a pressure value that is as consistently distributed as possible over the surface area in order to minimize variations in pressure between points at which the cable is gripped to avoid shearing or tearing of the surface fibers. An application of 13.6kg (30 lbs.) of force for example, can be achieved by 20.68 MPa (3000 psi) over an area of 0.0645 cm$^2$ (0.01 square inches), or by 2.068 MPa (300 psi) over an area of 0.645 cm$^2$ (0.10 square inches). The latter is a more optimal choice given the delicate nature of composite polymer cables such as described in the '246 patent. Consequently, a device capable of applying a relatively constant pressure P over a relatively large area of the cable surface resulting in an adequate value for the total integrated force, F, is desirable.

The above concerns are met by embodiments of the present surgical cartridges. Some embodiments of the present surgical cartridges comprise: a collar; and an insertable collet with a star-shaped cross section (insertable into the collar). The collets of the present designs can comprise several gripping fingers that move radially inward to grip the cable. The fingers can be configured to close in on the cable in a uniform manner, while maintaining as much contact area as possible between the collet and the cable. These embodiments may further be optimized by varying one or more of: the number of fingers and/or various properties of the fingers (e.g., the thickness of a sidewall defining a finger (finger wall) relative to the size of the cable and surgical cartridge with which the collar is designed to function). For example, an acceptable balance between insertion force (the force required to insert the collet into the collar to secure a cable) and pressure was achieved via a nine finger collet design. A seven finger design, although functional, required an unacceptably high crimping force to compress the fingers relative to the cable. A ten finger design could be functional using larger-diameter cables; however, for common cable (e.g., suture) diameters, the resulting wall thickness of the fingers for ten-finger configurations may become unworkably thin. Consequently, for certain of the present embodiments (e.g., those with collets including fingers defined by a sidewall), a delicate balance must be achieved between the number of fingers and the thickness of the finger wall.

More precisely, it is an objective of at least some embodiments of this disclosure to provide a gripping device capable of providing a firm, gentle, and/or generally (e.g., substantially) uniform pressure to a surgical cable comprised of a nylon or other polymeric material core covered by a polyethylene fiber braid.

It is a further objective of at least some embodiments of this disclosure to provide a gripping device that is biomedically compatible with the human body.

It is a still further objective of at least some embodiments of this disclosure to provide a gripping device capable of radial collapse in accordance with the shrinkage of a nylon- or other polymeric material-cored surgical cable undergoing tension while maintaining a firm grip throughout the range of variation in diameter of the cable during the process of tensioning the cable (e.g., between 100% and 87% of a relaxed or non-tensioned diameter).

It is a still further objective of at least some embodiments of this disclosure to provide a gripping device capable of maintaining a grip on the outer surface of a slippery, delicate cable, the grip being approximately (e.g., substantially) uniform along the length of a portion of the cable that is gripped by the device (e.g., along a majority of the length of the collet), and/or applying substantially equal pressures at contact regions disposed at substantially equiangular intervals around the circumference of the surgical cable.

It is a still further objective of at least some embodiments of this disclosure to provide a gripping device capable of maintaining a grip, yet not damaging, a delicate cable under high tension for a period of time adequate for the healing process to occur (e.g., a period of time for a fractured bone to heal).

Some embodiments of the present bio-compatible anchors comprise: a collet having a central longitudinal axis, a sidewall, a first end, a second end, and a length between the first end and the second end, the sidewall defining a plurality of ridges and valleys extending in a direction that is parallel in at least one plane to the central longitudinal axis; and a retaining collar having a passage configured to receive at least the first end of the collet; where the collet is configured to be inserted into the passage of the retaining collar to compress the sidewall toward the central longitudinal axis to provide a pressure to a surgical cable, the pressure being substantially equal at contact regions disposed at substantially equiangular intervals around the circumference of the surgical cable.

Some embodiments of the present bio-compatible anchors are configured to provide a substantially uniform pressure of 17.24 - 24.13 MPa (2500-3500 psi) to a surgical cable over a contact area of less than 0.323 $cm^2$ (0.05 square inches). In some embodiments, the contact area is substantially equally divided among contact regions disposed at substantially equiangular intervals around the circumference of the surgical cable.

Some embodiments further comprise: a collet having a central longitudinal axis, a sidewall, a first end, a second end, and a length between the first end and the second end, the sidewall defining a plurality of ridges and valleys extending parallel in at least one plane to the central longitudinal axis; and a retaining collar having a passage configured to receive at least the first end of the collet, where the collet is configured to be inserted into the passage of the retaining collar to compress the sidewall toward the central longitudinal axis. In some embodiments, the ridges and valleys of the sidewall define a plurality of fingers each having an inner end extending radially inward toward the central longitudinal axis, the fingers configured to be compressed inwardly toward the central longitudinal axis to apply pressure to the contact area of a cable disposed in the collet. In some embodiments, the pressure is substantially equal at contact regions disposed at substantially equiangular intervals around the circumference of the surgical cable. In some embodiments, the pressure is substantially uniform along a portion (e.g., a majority) of the length of the collet. In some embodiments, the relative distribution of the pressure remains substantially constant over cable diameter shrinkages of up to 15%.

In some embodiments of the present bio-compatible anchors the second end of the collet is larger than the first end of the collet. In some embodiments, the collet is tapered between the second end and the first end. In some embodiments, the collet is configured to be resilient. In some embodiments, at least one of the collet and collar comprises polymer. In some embodiments, the ridges and valleys of the sidewall define 7-11 radial fingers. In some embodiments, the sidewall has a thickness of 0.076 - 0.305 mm (0.003 - 0.012 inches). In some embodiments, at least one of the collar and the collet comprises a metallic alloy. In some embodiments, the metallic alloy comprises titanium. In some embodiments, the bio-compatible anchor is configured to provide the substantially uniform pressure to a surgical cable having a relaxed (non-tensioned diameter of between 0.25 millimeters (mm) and 2 mm (e.g., less than any one of, or between any two of: 0.3 mm, 0.5 mm, 0.075 mm, 1.0 mm, 1.5 mm, and/or 2 mm). In some embodiments, the biocompatible anchor is configured to substantially resist longitudinal movement of the surgical cable relative to the collet at tensions in the surgical cable of up to 600 Newtons (e.g., between 200 Newtons and 600 Newtons) or more (e.g., 1100 Newtons for a 2 mm cable). In some embodiments, collar comprises a first end, a second end, and a length between the first end and the second end, where the passage extends through and between the first end and the second end. In some embodiments, the collar is configured to receive the first end of the collet through the first end of the collar, and is configured to progressively compress the sidewall along the length of the collet as the first end of the collet is pressed into the passage toward the second end of the collar. In some embodiments, the first end of the collar includes an enlarged flange extending outwardly relative to the passage.

Some embodiments of the present the bio-compatible suture anchors further comprise a crimping tube coupled to the collar and parallel to a length of the collet. Some embodiments alternatively or additionally comprise a pair of opposing attachment tabs coupled to the collar (e.g., extending perpendicular to the length of the collet).

Some embodiments of the present installation tools for deployment of the present bio-compatible anchors comprise; an upper member configured to contact the collet of the bio-compatible anchor; and a lower member configured to contact the collar of the bio-compatible anchor and movable relative to the upper member to insert the collet into the collar. In some embodiments, the installation tool is configured to grip a surgical cable extending through the bio-compatible anchor when the bio-compatible anchor is in contact with the upper and lower members of the installation tool. Some embodiments further comprise: a lever including a passageway configured to receive the surgical cable; and a tension member configured to resist withdrawal of the surgical cable from the passageway to maintain a tension in the surgical cable. In some embodiments, the upper member is pivotally coupled to the lower member (e.g., in a scissor-like configuration). In some embodiments, the lower member comprises a forked portion configured to receive the collar, the upper member comprises a forked portion configured to receive the surgical cable.

Some embodiments of the present kits comprise: an embodiment of the present bio-compatible anchors; and a package within which the bio-compatible anchor is disposed; where the bio-compatible anchor is sterile. Some embodiments of the present kits comprise: an embodiment of the present bio-compatible anchors; and an embodiment of the present installation tools. Some embodiments of the present kits comprise: an embodiment of the present bio-compatible anchors; and a washer having an inner transverse dimension that is greater than an outer transverse dimension of a portion of the collar between the first end and the second end of the collar, and smaller than an outer transverse dimension of the flange. Some embodiments further comprise an embodiment of the present installation tools; and/or a package within which the bio-compatible anchor is disposed; where the bio-compatible anchor is sterile.

Some embodiments of the present methods (e.g., of deploying a surgical cable to stabilize a fractured bone) comprise: inserting the collet of an embodiment of the present bio-compatible anchors into the passage of the collar of the bio-compatible anchor while a surgical cable is disposed in the collet, such that the sidewall of the collet is compressed to grip the surgical cable. Some embodiments further comprise: tensioning the surgical cable prior to inserting the collet. Some embodiments further comprise: inserting the surgical cable into the collet prior to inserting the collet. Some embodiments further comprise: disposing the lower member of an embodiment of the present installation tools in contact with the collar; disposing the upper member of the installation tool in contact with the collet; where inserting the collet comprises moving the upper member relative to the lower member to push the collet and the collar together. In some embodiments, the surgical cable is in contact with the fractured bone. In some embodiments, the surgical cable extends through an aperture in the bone. Some embodiments further comprise disposing the collar in the aperture in the bone. In some embodiments, the bio-compatible anchor comprises a crimping tube coupled to the collar and parallel to the length of the collar, and the method further comprises: crimping an end of the surgical cable in the crimping tube and/or encircling two or more pieces of a fractured bone with a cable that is secured in the crimping tube. In some embodiments, the bio-compatible anchor comprises a pair of opposing attachment tabs coupled to the collar and extending perpendicular to the length of the collar, the method further comprising: securing the attachment tabs to a bone via one or more fasteners extending through the attachment tabs into the bone.

Some embodiments include a bio-compatible high tension suture anchor capable of providing a consistently uniform pressure of 17.24 - 24.13 MPa (2500-3500 psi) over an area measuring less than 0.323 $cm^2$ (.05 square inches) is disclosed. The bio-compatible high tension suture anchor may further comprise a truncated, hollow, conical cylinder having a length, a radius, and a cylindrical wall. The cylindrical wall may be comprised of a regular series of ridges and valleys parallel to the length of the conical cylinder. It may also comprise a retaining collar operable for progressively compressing the radius of the conical cylinder upon insertion of the conical cylinder within the retaining collar. The regular series of ridges and valleys may define a radial arrangement of

compressive fingers, the fingers being operable for radially uniform compression of a cable inserted within the hollow space of the conical cylinder. The radially uniform compression may be approximately invariant over the length of the conical cylinder. The radially uniform compression may be consistent over cable diameter shrinkages of up to 15%. The bio-compatible high tension suture anchor may have 7-11 radial fingers. The radial fingers may have a wall thickness of 0.076 - 0.305 mm (0.003 - 0.012 inches). The high tension suture may be made of a metallic alloy. The metallic alloy may be titanium.

In some embodiments, the bio-compatible high tension suture anchor may also have a crimping tube integral with the suture anchor and parallel to the length of the conical cylinder. Alternatively, it may include a pair of opposing attachment tabs integral with the suture anchor and perpendicular to the length of the conical cylinder.

An exemplary installation tool for deployment of the high tension suture anchor is also described and claimed. The tool comprises an upper forked member and a lower forked member. The upper forked member and the lower forked member are configured in a scissor-like fashion. The lower forked member is operable for securing and positioning the high tension suture anchor. The tool also comprises an auxiliary lever operable for gripping a surgical cable threaded through the high tension suture anchor and a positioning conduit operable for positioning and tightening a surgical cable to the desired level of tension. The tool further comprises a tension retaining member operable for maintaining the desired level of tension on the surgical cable throughout the deployment process.

Some embodiments of the present methods of deploying a surgical cable within a fractured bone comprise the steps of:

1) Presenting any one of the above described high tension suture anchors,
2) Presenting an installation tool as previously described
3) Positioning the anchor within the tool, thereby forming a tooled anchor assembly,
4) Threading a surgical cable through the tooled anchor assembly,
5) Pressing or otherwise inserting the anchor assembly into a surgically drilled bone aperture while pulling the surgical cable taut,
6) Squeezing the upper and lower forked members of the installation tool together, while urging the conical cylinder of the anchor into the throat of its retaining collar, thereby gripping and securing the inserted surgical cable,
7) Releasing the tension on the surgical cable,
8) Sliding the upper and lower fork members away from the anchor assembly, and
9) Cutting the extraneous length of the surgical cable.

Some embodiments of the present methods of employing cerclage to an assembly of fractured bones using a surgical cable comprise the steps of:

1) Presenting a high tension suture anchor having an integrated crimping tube,
2) Presenting an installation tool as previously described,
3) Positioning the anchor within the tool, thereby forming a tooled anchor assembly,
4) Crimping one end of the surgical cable in the crimping tube,
5) Encircling the assembly of fractured bones with the free end of the surgical cable,
6) Threading the free end of the surgical cable through the tooled anchor assembly,
7) Squeezing the upper and lower forked members of the installation tool together, while urging the conical cylinder of the anchor into the throat of its retaining collar, thereby gripping and securing the inserted surgical cable,
8) Releasing the tension on the surgical cable,
9) Sliding the upper and lower fork members away from the anchor assembly, and
10) Cutting the extraneous length of the surgical cable.

Some embodiments of the present methods of deploying a surgical cable to the surface of a fractured bone comprise the steps of:

1) Presenting a high tension suture anchor with opposing tabs,
2) Presenting an installation tool as previously described,
3) Positioning the anchor within the tool, thereby forming a tooled anchor assembly,
4) Threading a surgical cable through the tooled anchor assembly,
5) Pressing the anchor assembly against the surface of the fractured bone such that the opposing tabs lie flat against the bone,
6) Screwing the anchor assembly to the fractured bone using the opposing tabs,
7) Pulling the surgical cable taut,
8) Squeezing the upper and lower forked members of the installation tool together, while urging the conical cylinder of the anchor into the throat of its retaining collar, thereby gripping and securing the inserted surgical cable,

9) Releasing the tension on the surgical cable,

10)Sliding the upper and lower fork members away from the anchor assembly, and

11)Cutting the extraneous length of the surgical cable.

The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified (and includes what is specified; e.g., substantially 90 degrees includes 90 degrees and substantially parallel includes parallel), as understood by a person of ordinary skill in the art. In any disclosed embodiment, the terms "substantially," "approximately," and "about" may be substituted with "within [a percentage] of" what is specified, where the percentage includes .1, 1, 5, and 10 percent.

Further, a device or system that is configured in a certain way is configured in at least that way, but it can also be configured in other ways than those specifically described.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, an apparatus that "comprises," "has," "includes" or "contains" one or more elements possesses those one or more elements, but is not limited to possessing only those elements. Likewise, a method that "comprises," "has," "includes" or "contains" one or more steps possesses those one or more steps, but is not limited to possessing only those one or more steps.

Any embodiment of any of the apparatuses, systems, and methods can consist of or consist essentially of - rather than comprise/include/contain/have - any of the described steps, elements, and/or features. Thus, in any of the claims, the term "consisting of" or "consisting essentially of" can be substituted for any of the open-ended linking verbs recited above, in order to change the scope of a given claim from what it would otherwise be using the open-ended linking verb.

The feature or features of one embodiment may be applied to other embodiments, even though not described or illustrated, unless expressly prohibited by this disclosure or the nature of the embodiments.

Details associated with the embodiments described above and others are described below.

## BRIEF DESCRIPTION OF ITEMS IN THE FIGURES

10 - cable (prior art)
14 - polymeric material core (prior art)
15 - high strength UHMW polyethylene fiber braid (prior art)
101 - surgical cartridge
102 - bone fragments
103 - surgical cable
104 - optional washer
105 - outer bone aperture for surgical cable (103)
201 - retaining collar
202 - collet having a star-shaped cross section
203 - central void or passageway through collet
204 - passage of the collar
205 - first end of the collar
206 - second end of the collar
207 - length of the collar
208 - enlarged flange of the collar
209 - narrow portion of the collar
210 - primary portion of the collar
211 - central longitudinal axis of the collet
212 - sidewall of the collet
213 - first end of the collet
214 - second end of the collet
21 5 - length of the collet
216 -ridges of the collet sidewall
217 valleys of the collet sidewall
302 - outer contacting surface of cable (103)
303 - inside surface of the collet sidewall
304 - outside surface of the collet sidewall
305 - collet finger
306 - inner end of collet finger
401 - radial cross section of uncompressed collet with cable (103) inserted
402 - radial cross section of collet compressed around cable (103)
501 - radial plane near top of collet (202)
502 - radial plane approximately one third collet length from the top of collet (202)
503 - radial plane approximately two thirds collet length from the top of collet (202)
504 - radial plane near bottom of collet (202)
700 - exemplary installation tool
701 - upper member
702 - lower member
703 - forked portion of the lower member
704 - forked portion of the upper member
705 - lever of the installation tool
706 - passageway of the tool for receiving a surgical cable (103)
707 - tension member
800 - installed suture anchor (101)
801 - simplified embodiment of installation tool with lever (705) omitted
901 - cerclage cartridge
902 - crimping tube of cerclage cartridge
903 - collar of cerclage cartridge
1001 - tabbed cartridge
1002 - collar of tabbed cartridge

1003 - attachment tabs of tabbed cartridge

## BRIEF DESCRIPTION OF THE FIGURES

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. The figures are drawn to scale (unless otherwise noted), meaning the sizes of the depicted elements are accurate relative to each other for at least the embodiments depicted in the figures.

Figure 1: An illustration of the scenario wherein the depicted embodiment (101) of the present surgical cartridges is used to secure bone fragments (102). Figure 1A indicates an overall view of two of the surgical cartridges (101) each positioned to be affixed to a different end of the surgical cable (103) that extends through apertures (105) in an outer surface of two pieces or fragments (102) of the bone to be stabilized and/or secured relative to each other. Figure 1B shows an expanded view of the dotted oval region in Figure 1A.

Figure 2: The surgical cartridge (101) is shown in greater detail. The collet (202) has a star-shaped cross section and surrounds a central void (203), with at least a portion of the collet (202) surrounded or enclosed by the retaining collar (201), as shown.

Figure 3: The star-shaped collet (202) is shown in greater detail. Fig. 3A indicates a perspective view of the collet (202), and Figs. 3B-3E indicate end views of a second end (214) of the collet (202) with cable (103) inserted or disposed in the central void (203). The inside (303) and outside (304) surfaces of the wall of each collet finger (305) are indicated in more detail in Figs. 3D and 3E, as well as the contacting outer surface (302) of the cable (103).

Figure 4: Fig. 4A indicates an end view of the second end (214) of the star-shaped collet (202) in an uncompressed state (401) with an inserted surgical cable (103). Fig. 4C indicates a compressed state (402) of the star-shaped collet (202).

Figs. 4B and 4D notionally indicate the force between the collet (202) and surgical cable (103) according to angular position around the outer surface (302) of the cable in Figs. 4A and 4B, respectively.

Figure 5: Figs. 5A-5B notionally indicate the force between the collet (202) and surgical cable (103) according to angular position around the outer surface (302) of the cable for several positions (501)-(504) along the collet (202) length.

Figure 6: Prior art cable (10) showing inner core (14) and outer braid (15).

Figure 7: Installation tool (700) for installation of surgical cartridge (101) and surgical cable (103) inserted therein.

Figure 8: Installation tool (700) detailing features and use of upper and lower members (701), (702).

Figure 9: Cerclage cartridge (901) with surgical cable (103) installed therein.

Figure 10: Front and side views showing fractured flat bones (102) held in compression by a surgical cable (103). Each end of the cable is secured by a tabbed cartridge (1001) screwed into the flat bone segments.

## DETAILED DESCRIPTION OF ILLUSTRATIVE EMBODIMENTS

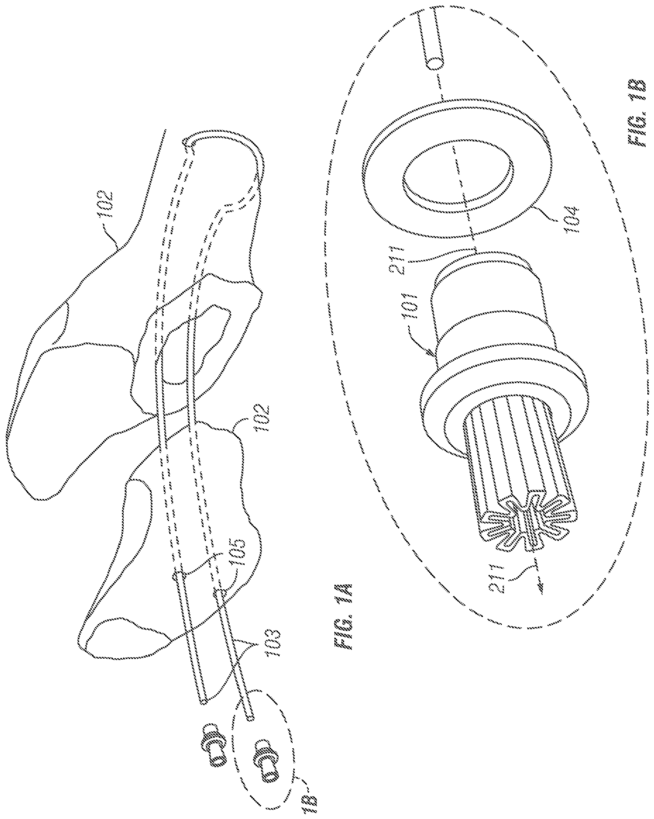

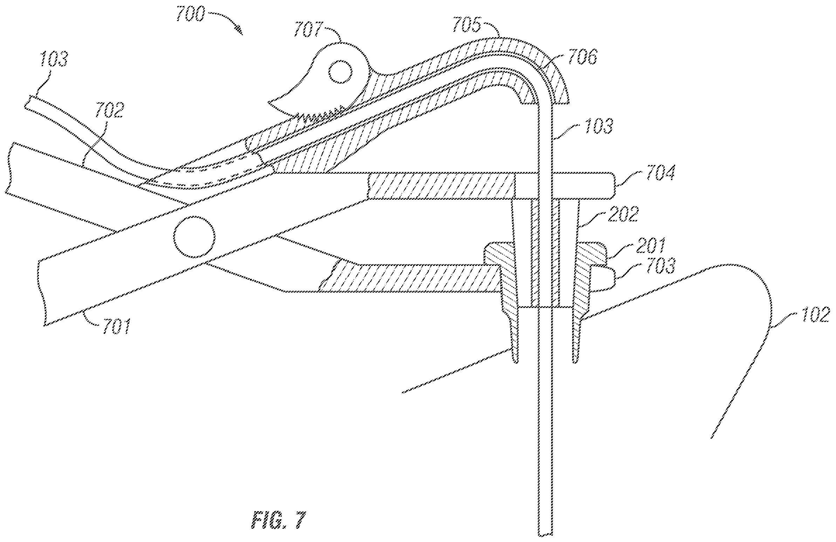

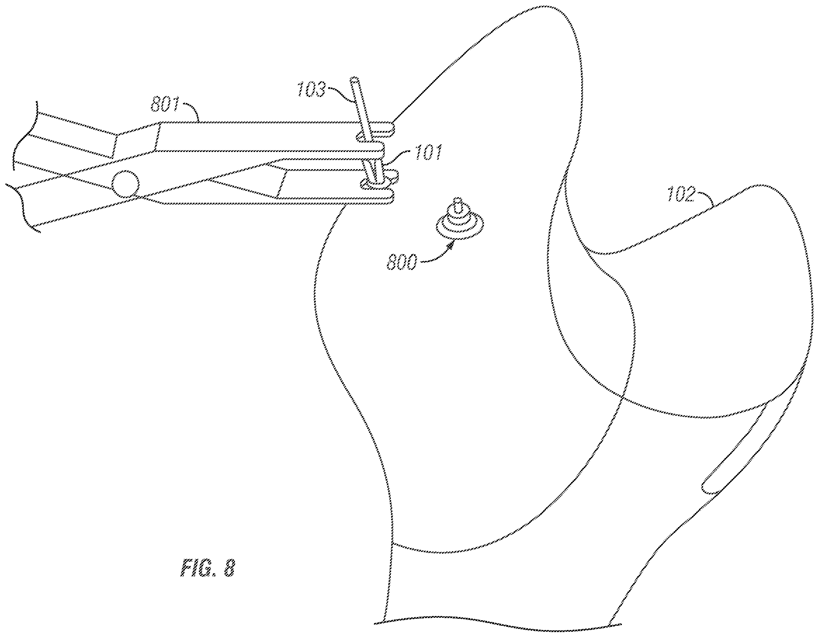

Figure 1 illustrates the use of an embodiment of the present surgical cartridges. In the exemplary implementation shown, a surgical cable (103) has been threaded through bone fragments (102) via apertures (105), as well as the central voids (203) of the collets (202) of the two surgical cartridges (101). A tool (e.g., as in Figs. 7-8) may then be used to draw up, apply the required tension to the surgical cable (103), and seat each surgical cartridge (101) into the outer bone aperture (105). An optional washer (104) may be interspersed or disposed between each surgical cartridge (101) and the respective outer bone aperture (105).

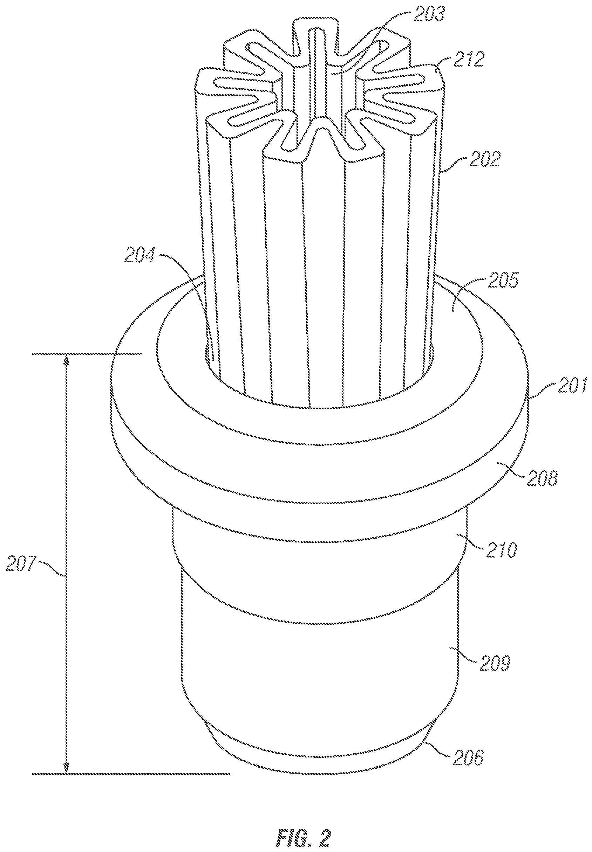

Figure 2 shows the surgical cartridge (101) in greater detail. The retaining collar (201) holds the star-shaped collet (202) and the central void (203) accommodates the surgical cable (103). In the embodiment shown, the retaining collar (201) includes a passage (204) configured to receive at least the first end (213) of the collet (202). In this embodiment, the collar (201) has a first end (205), a second end (206), and a length (207) between the first end (205) and the second end (206). As shown, the passage (204) extends through and between the first end (205) and the second end (206). In the embodiment shown, the first end (205) of the collar (201) includes an enlarged flange (208) extending outwardly relative to the passage (204). In this embodiment, the collar includes a narrow portion (209) adjacent to the second end (206) having a transverse dimension (e.g., diameter) that is smaller than a transverse dimension of a primary portion (210) that is between the narrow portion and the first end (205) of the collar (201). In the embodiment shown, the outer surface of the collar includes three circular cylindrical portions (narrow portion (209), primary portion (210), and flange (208)) with successively increasing diameters. In other embodiments, the collar can have any suitable outer shape (e.g., square, hexagonal, octagonal, etc.) and/or may omit the narrow portion (209).

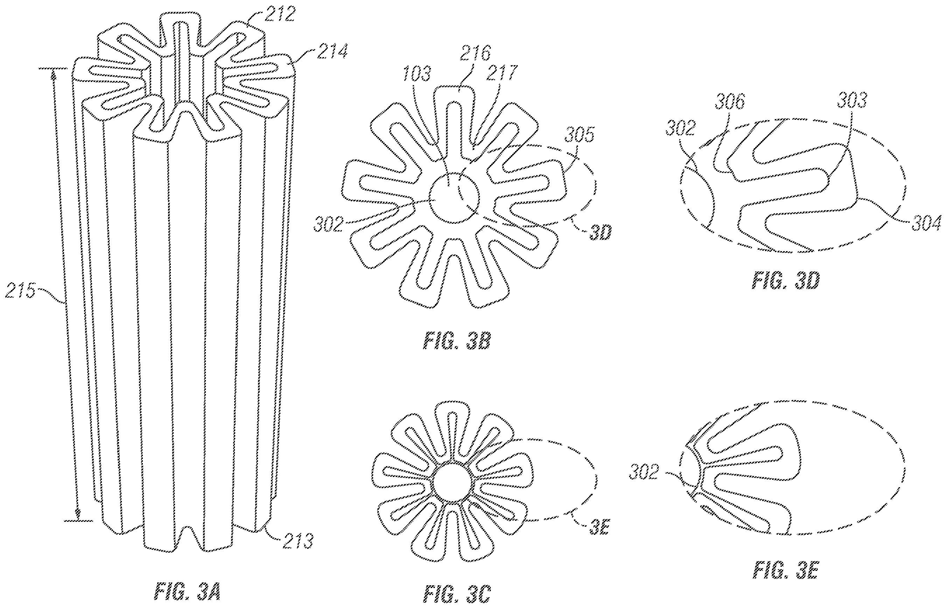

The depicted embodiment (202) of the present star-shaped collets is detailed in Figs. 3A-3E. The perspective view shown in Fig. 3A illustrates the lengthwise features on the exterior of the collet (202). Figs. 3B-3E illustrate varying stages of deployment of the collet onto the inserted cable (103). Fig. 3B, along with the enhanced insert of Fig. 3D, indicates the offset position of the inside surface of the collet wall (303) with respect to the contacting surface (302) of the cable (103) for one collet finger (305).

In the embodiment shown, cartridge (101) is configured to provide a substantially uniform pressure (e.g., of 17.24 - 24.13 MPa (2500-3500 psi)) to a surgical cable (e.g., 103) over a contact area (e.g., of less than 0.323 cm$^2$ (0.05 square inches)) between the collet (202) and the outer surface (302) of the surgical cable.

In this embodiment, the collet (202) has a central longitudinal axis (211), a sidewall (212), a first end (213), a second end (214), and a length (215) between the first end (213) and the second end (214). As shown, in this embodiment, the sidewall (212) defines a plurality of ridges (216) and valleys (217) that extend in the general direction of (e.g., parallel, in at least one plane, to) the central longitudinal axis (211). As shown, the ridges (216) are farther from central longitudinal axis (211) than are valleys (217). As shown in Figs. 1B and 2, the collet (202) is configured to be inserted into the passage (204) of the retaining collar (201) to compress the sidewall (212) toward the central longitudinal axis (211) to provide a pressure to a surgical cable (103). In this embodiment, the collet (202) and/or collar (201) is configured such that the pressure is substantially equal at contact regions (e.g., adjacent to the valleys (217)) disposed at substantially equiangular intervals around the circumference of the surgical cable, as shown. In some embodiments, the collet (202) is configured to be resilient. For example, in some embodiments, the collet (202) comprises a polymer. In some embodiments, the collet (202) and/or the collar (201) comprises a metallic alloy (e.g., titanium).

In some embodiments, the second end (214) of the collet (202) is larger (e.g., has a larger transverse dimension in the uncompressed state of the collet) than the first end (213) of the collet (202). For example, in the embodiment shown, the outer profile (defined by the outermost portions of the collet ridges (216) and, more particularly) of the outside surface (304) of the collet sidewall (305) at the ridges (216)) the collet (202) is tapered (e.g., linearly, as shown in Figure 3A) between the larger second end (214) and the smaller first end (213). In some embodiments, the outer profile is tapered at an angle of between three (3) and nine (9) degrees (e.g., six (6) degrees, as in the depicted embodiment) relative to the longitudinal axis (211) of the collet. In the embodiment shown, the inner profile (defined by valleys (217) and, more particularly, by inner ends (306) of collet fingers (305)) of the collet (202) is not tapered (valleys (217) are parallel to each other) such that the inner profile of the collet (202) matches the outer profile of the cable. In this embodiment, channel (204) of collar (201) is tapered (e.g., linearly, as shown in Figure 3A) between the larger first end (205) and the smaller second end (206) to correspond to the outer profile of the cable, such that as the collet (202) is inserted into the collar (201), the valleys (217) of the collet (202) are pushed inward into the cable but also maintain their parallel configuration so as not to pinch the cable.

In the embodiment shown, the ridges (216) and the valleys (217) define a plurality of fingers or collet fingers (305) each having an inner end (306) extending radially inward toward the central longitudinal axis (211). As shown in Figs. 3C and 3E, the fingers (305) are configured to be compressed inwardly toward the central longitudinal axis (211) to apply pressure to the contact area of (e.g., the outer surface (302) of) a cable (103) disposed in the collet (202). In some embodiments, the ridges (216) and the valleys (217) of the sidewall (212) define between 7 and 11 (e.g., radial) fingers (305). For example, the embodiment (202) of the collet shown includes nine fingers (305) and is configured for use with a cable having a relaxed or non-tensioned diameter of 0.813 mm (0.032 inches). In some embodiments, the sidewall (212) has a thickness of between 0.051 - 0.305 mm (0.002 and 0.012 inches). For example, in the embodiment shown, the sidewall (212) has a thickness of 0.004 inches. In the embodiment shown, the sidewall (212) of the collet (202) is configured to be progressively compressed along the length (215) of the collet (202) as the first end (213) of the collet (202) is pressed into the passage (204) of the collar (201) toward the second end (206) of the collar (201). In the embodiment shown, the inner end (306) of each collet finger (305) is slightly concave. In other embodiments, the inner end of each collet finger may be flat or convex.

In Figs. 3B and 3D, the collet (202) is uncompressed, and, thus, the cable may slide freely through the central void (203) of the collet (202). By contrast, Fig. 3C, along with the enhanced insert of Fig. 3E, indicate collet (202) in a compressed (deployed) state. In the compressed state, the inside surface of the collet wall (303) is pressed tightly against the contacting surface (302) of the cable (103), thereby preventing movement of the cable (103) along the central void (203) of the collet (202). The ability of the collet (202) to be compressed in this manner while maintaining physical integrity is determined by both the collet (202) material as well as the thickness of the collet wall, the (e.g., approximate and/or average) distance between its inner (302) and outer (303) surfaces. Both factors are important design parameters, and may be varied for various types and/or sizes of surgical cables with which cartridge or anchor (101) is configured to function.

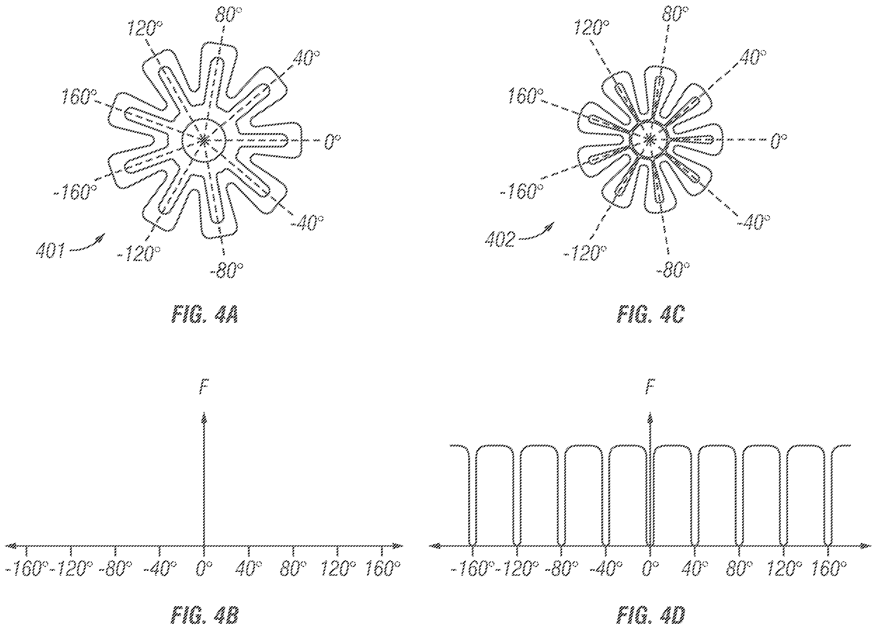

End views of the second end (206) of the star-shaped collet (202) with inserted surgical cable (103) is again shown in an uncompressed state in Figs. 4A-4B, with the corresponding illustrations for the compressed state shown in Figs. 4C-4D. Figs 4A and 4C are simply re-rendered versions of Figs. 3B and 3C with angular reference axis superimposed thereon. In the embodiment shown, the contact area between the collet (202) and the outer surface (302) of the surgical cable (103) is substantially equally divided among contact regions (between each finger (305)) and outer surface (302) disposed at substantially equiangular intervals (e.g., angular intervals of 40 degrees, as shown) around the circumference of the surgical cable

Figs. 4B and 4D show a notional depiction of applied pressure, P, versus circumferential angle for Figs. 4A and 4C, respectively. In the uncompressed state, Figs. 4A-4B, the pressure exerted by the collet (202) on the cable (103) is zero for all angles since there is no contact between the inside surface of the collet wall (303) and the contacting surface (302) of the cable (103). By contrast, the notional depiction of applied pressure, P, versus angle shown in Fig. 4D for the compressed state depicted in Fig. 4C, indicates a regular non-zero behavior. For example, the pressure is minimal at 0°, ±40°, ±80°, ±120°, and ±160° because, at these points the inside surface of the collet wall (303) folds away from the contacting surface (302) of the cable (103). Alternatively, the pressure is maximal at 20°, ±60°, ±100°, ±140°, and 180° since these are points of maximal compression. As indicated, the collet (202) is configured to provide pressure substantially equally at the contact regions between the fingers (305) of the collet (202) and the outer surface (302) of the surgical cable (103). In the embodiment shown, the collet (202 is further configured to compress the surgical cable (103) with sufficient force to retain the surgical cable (103) against (e.g., resist longitudinal movement of the surgical cable relative to the collet at) tensions of up to 100 Newtons, 200 Newtons, or more (e.g., 800 Newtons, 1100 Newtons) in the cable (depending on cable and/or collet size) and/or that may result in the diameter of the surgical cable (103) shrinking by as much as 15% of its relaxed (non-tensioned) diameter, and/or such that the relative distribution of the pressure (e.g., between and among contact regions) remains substantially constant over cable diameter shrinkages of up to 15%. The collet (202) and/or the collar (201) can be configured to provide the substantially uniform pressure to a surgical cable or suture.

Of course, the detailed behavior of a working collet (202) is determined primarily by the shape and number of collet fingers (305). However, an optimal number of fingers is a design parameter that must be determined in balance with the parametrical design consideration of wall thickness. As earlier stated, a nine finger design appears to function well.

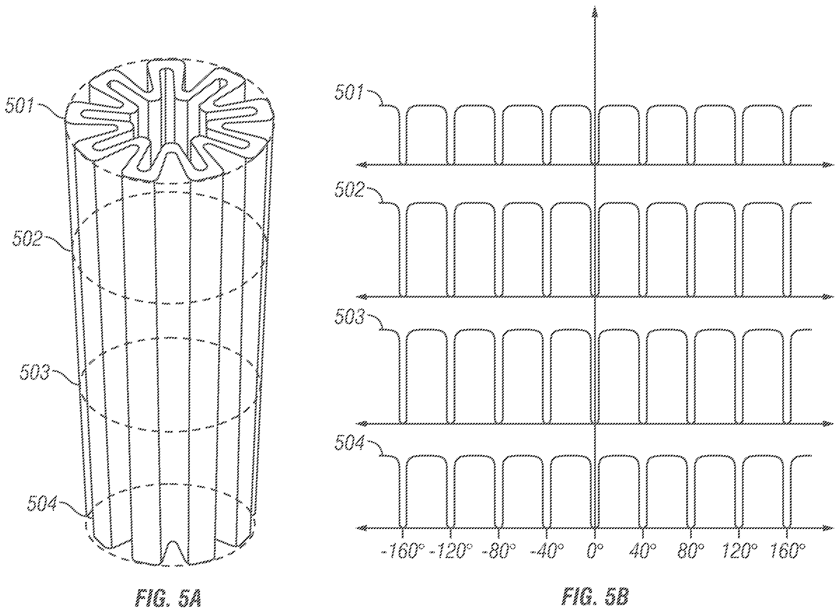

Figure 5 shows a notional depiction of applied pressure, P, versus angle for various locations (501)-(504) along the length of the collet (202). As shown, the radial distribution of pressure is substantially the same (the magnitude of pressure at each finger is substantially the same) at each point along the length of the collet (202), even though the magnitude of pressure (at points 501 and 504) at either of end of the collet (202) are lower than those (at point 502 and 503) in longitudinal middle portions of the collet (202). For example, the pressure distribution shown in Figure 5B can be achieved by enlarging (e.g., tapering or flaring outward) a portion of the inner profile (defined by valleys (217)) of the collet (202) near the first end (213) of the collet (202) such that the first end (213) does not grip the cable as strongly as the remainder of the collet (202), as indicated at point 504 of Figure 5B. In this embodiment, the second end (214) of the collet (202) can extend out of the first end (205) of the collar (201) when gripping a cable of a given diameter under expected operating tensions (e.g., 200 Newtons) such that the second end (214) also does not grip the cable as the remainder of the collet (202), as indicated at point 501 of Figure 5B. Although a slight variation (shown exaggerated for clarity) in maximal pressure is notionally indicated in the figure, this deviation should be made as minimal as possible (e.g., less than any one of, or between any two of: 5%, 10%, 15%, 20%, and/or 25%). In other embodiments, the collar (201) and collet (202) are configured such that the pressure is substantially uniform along the length (215) of the collet (202).

If concerns discussed above have been adequately addressed, the total force exerted on the cable (103) should be spread or distributed over as large an area as possible so that the applied pressure for any given unit surface area is not unduly high, which might otherwise risk tearing and/or shearing of the delicate fibers covering the cable's outer surface. Consider a length of the cable (10) of Figure 6. The braided fibers (15) wind around the core (14) in a helical fashion. Some fibers wind to the left while others wind to the right; both groups knit together to make up the braid. The relatively broad fingers of the collet capture this braided arrangement of crossed helical fibers, consistently engaging them in a balanced manner about the core circumference as the cable shrinks under tension.

Figures 7-8 illustrate an exemplary installation tool (700) for deployment of the surgical cartridge (101) and with a surgical cable (103) inserted therein. The installation tool (700) includes: an upper member (701) configured to contact the collet (202) of the surgical cartridge (101); and a lower member (702) configured to contact the collar (201) and movable relative to the upper member (701) to insert the collet (202) into the collar (201). For example, in the embodiment shown, the lower member (702) includes a forked portion (703) configured to receive the collar (201), as shown, and the upper member (701) includes a forked portion (704) configured to receive the surgical cable (103), as shown. For example, the lower forked portion (703) can receive primary portion (210) of the collar (201) and contact the enlarged flange (208) of the collar (201), and upper forked portion (704) can receive the surgical cable (103) and contact the second end (214) of the collet (202), as shown, such that the tool (700) can effectively grip the lower surface of the flange (208) of the retaining collar (201) and the upper surface of an inserted collet (202) to compress the collet and collar relative to each other.

In some embodiments, the tool (700) is configured to grip a surgical cable (103) extending through the bio-compatible anchor or cartridge (101) when the bio-compatible anchor is in contact with the upper and lower members (701 and 702) of the installation tool (700). For example, in the embodiment shown, the tool (700) also comprises a lever (705) that includes a passageway (706) configured to receive the surgical cable (103), as shown, and through which the surgical cable (103) can be threaded and tightened to the required level of tension. In this embodiment, the tool (700) also comprises a tension member (707) that is configured to resist withdrawal of the surgical cable (103) from the passageway (706) to maintain a tension in the surgical cable (103), such as, for example, during insertion of the collet (202) into the collar (201) to compress the collet (201) to grip the surgical cable (103). The upper member (701) and lower member (702) thus not only allow the cartridge assembly to be properly positioned, but also facilitate sliding the collet (202) into the retaining collar (201), thereby providing a gently increasing and uniform grip on a tightened surgical cable (103).

Some embodiments of the present kits comprise one or more of: an embodiment of the present bio-compatible anchors or cartridges (e.g., 101); and an embodiment of the present installation tools (e.g., 700). Some embodiments further comprise a package within which the bio-compatible anchor or cartridge is disposed (e.g., sealed). In some embodiments, the bio-compatible anchor or cartridge is sterile. In embodiments of the present kits in which the collar (e.g., 201) includes an enlarged flange (e.g., 208), the kit can further comprise: a washer having an inner transverse dimension that is greater than an outer transverse dimension of a portion (e.g., the primary portion (210)) of the collar between the first end and the second end of the collar, and smaller than an outer transverse dimension of the flange.

Some embodiments of the present methods (e.g., of deploying a surgical cable to stabilize a fractured bone) comprise: inserting the collet of one of the present bio-compatible anchors or cartridges (e.g., 101) into the passage (e.g., 204) of the collar while a surgical cable (e.g., 103) is disposed in the collet (e.g., 202), such that the sidewall (e.g., 212) of the collet is compressed to grip the surgical cable. Some embodiments further comprise: tensioning the surgical cable prior to inserting the collet. Some embodiments further comprise: inserting the surgical cable into the collet prior to inserting the collet. Some embodiments further comprise: disposing the lower member (e.g., 702) of an installation tool (e.g., 700) in contact with the collar (e.g., 201); disposing the upper member (e.g., 701) of the installation tool in contact with the collet (e.g., 202); and inserting the collet comprises moving the upper member relative to the lower member to push the collet and the collar together. In some embodiments, the surgical cable is in contact with the fractured bone (e.g., the surgical cable extends through an aperture (e.g., 105) in the bone). Some embodiments, further comprise: disposing the collar in the aperture in the bone. For example, installation of the surgical cartridge (101) with surgical cable (103) threaded therein may occur as follows:

1. A surgical cable (103) extending around and/or through a bone (102) (e.g., via one or more apertures (105)) is threaded through the surgical cartridge (101) assembly (e.g., through the collet (202)),

2. The surgical cartridge (101) (e.g., the enlarged flange (208) of the collar (201) is pressed against the bone as the surgical cable(103) is pulled taut (tensioned),

3. The upper member (701) and lower member (702) are squeezed together, urging the collet (202) into the passage (204) of retaining collar (201), thereby compressing the fingers (305) of the collet (202) in on the surgical cable (103) inserted therein.

4. The tension is released in the portion of the surgical cable (103) extending past the cartridge (101) (on the side of the cartridge opposite the bone),

5. The upper and lower members (701 and 702) are slid away from the surgical cartridge (101) assembly, and

6. Any extraneous cable length is cut.

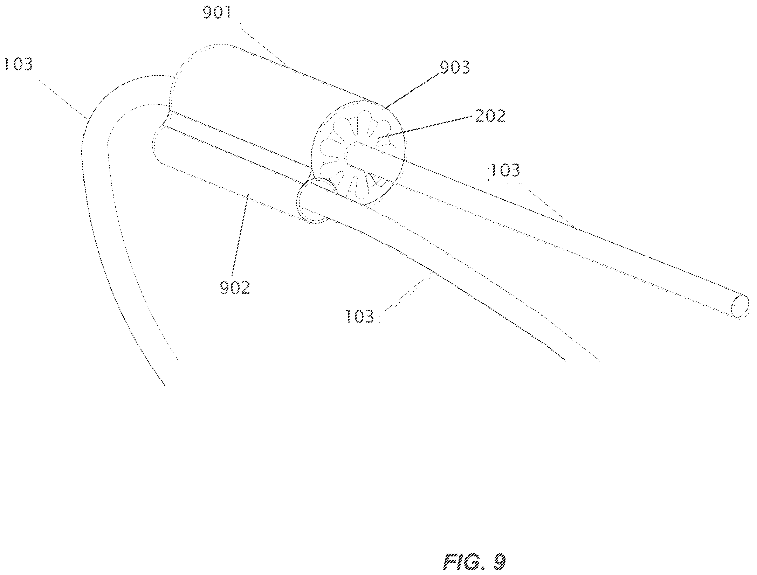

A cerclage cartridge (901) with an integrated attachment site is shown in Figure 9. In the embodiment shown, a first end of the surgical cable (103) can be crimped by conventional means into a crimping tube (902) that is coupled to (e.g., unitary with) the collar (903) since, in its untensioned state, the surgical cable (103) is relatively soft and bulky. In the embodiment shown, The free end is then positioned as required or otherwise desired and the cable pulled taught. In a tensioned state, the diameter of the surgical cable (103) shrinks and the cable as a whole becomes taut and slippery, requiring the distributed radial compression afforded by the collar (903) and collet (202) to effectively secure the remaining end of the cable. Consequently, the cerclage cartridge (901) is particularly well suited to address attachment of the surgical cable (103) in the depicted arrangement. In some embodiments, the cerclage cartridge (901) includes a length (e.g., greater than any one of, or between any two of: 305 mm, 457 mm, 610 mm, 762 mm and/or 914 mm (12 inches, 18 inches, 24 inches, 30 inches and/or 36 inches)) of cable (101) having a portion (e.g., adjacent to an end of the cable) that is crimped within the crimping tube (902) and/or otherwise secured to collar (903). While the cerclage cartridge (901) may be suitable for certain cerclage techniques and implementations, it may also be used for implementations such as the one depicted in Figure 1A to secure two ends of a surgical cable that extends through holes drilled through a fractured bone.

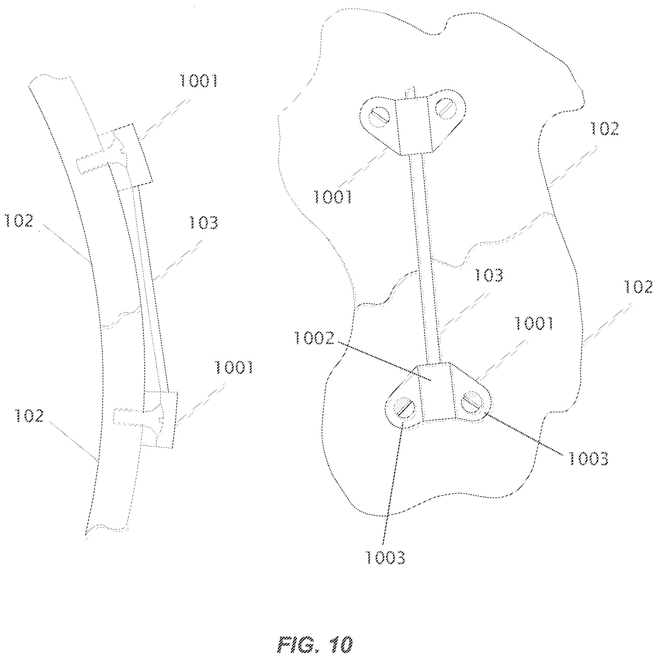

Figure 10 shows front and side views of a fractured flat bone (102) held in compression by a surgical cable (103). Tabbed cartridges (1001) screwed into the flat bone segments secure each end of the surgical cable (103). In this embodiment, each cartridge (1001) includes a collar (1002) having a passage (in which cable 103 is shown secured by a collet 202) that is similar to passage (204) of collar (201). This embodiment further includes a pair of opposing attachment tabs (1003) coupled to the collar (1002) and extending perpendicular to the length of the collar.

Alternate embodiments envisioned but not shown include one or more surgical cartridges (101) integrated with other attachment devices such as a bone plate. Indeed, reconstruction techniques may include one or several surgical cartridges securing a network of surgical cables (103) and other attachment devices. In addition, the surgical cartridge (103) can be used to secure other medical tethers, such as (for instance), spider silk.

Recalling the objectives stated in the Summary section of this disclosure, the present embodiments of surgical cartridges or anchors provide a bio-medically compatible gripping device capable of radial collapse in accordance with the shrinkage of nylon or other polymeric material cored surgical cable undergoing tension while maintaining a firm grip throughout the process of tensioning the surgical cable and certain changes in diameter in the surgical cable that may result from the tensioning. Moreover, the present embodiments of surgical cartridges and/or anchors can provide gripping devices capable of maintaining a grip on the outer surface of a slippery delicate cable, the grip being approximately (e.g., substantially) uniform among contact regions disposed (e.g., at substantially equiangular intervals) around the circumference of the cable at one or more points along the length of the cable. For example, at a first longitudinal point along the length of a gripped portion of the cable, the radial distribution of pressure is substantially uniform; and at a second longitudinal point along the length of a gripped portion of the cable, the radial distribution of pressure is substantially uniform; even if the magnitude of pressure is greater at the first longitudinal point than at the second longitudinal point (in some embodiments, the distribution of pressure is also constant along a majority of the length of a gripped portion of the cable, such as, for example, along the entire length of the collet (202)). The present embodiments of surgical cartridges or anchors also provide gripping devices capable of maintaining a grip, yet not damaging, a delicate cable under high tension for a period of time adequate for as the healing process to occur.

While several illustrative embodiments have been shown and described, numerous variations and alternate embodiments will occur to those skilled in the art, and can be made without departing from the scope of the invention as defined in the appended claims. The above specification and examples provide a complete description of the structure and use of exemplary embodiments. Although certain embodiments have been described above with a certain degree of particularity, or with reference to one or more individual embodiments, those skilled in the art could make numerous alterations to the disclosed embodiments without departing from the scope of this invention. As such, the various illustrative embodiments of the present devices and kits are not intended to be limited to the particular forms disclosed. Rather, they include all modifications and alternatives falling within the scope of the claims, and embodiments other than the one shown may include some or all of the features of the depicted embodiment. For example, the present collets can include any suitable number of ridges, valleys, and/or fingers. For example, components may be combined as a unitary structure, and/or connections may be substituted (e.g., in some embodiments, the present collets may be unitary with the present collars). Further, where appropriate, aspects of any of the examples described above may be combined with aspects of any of the other examples described to form further examples having comparable or different properties and addressing the same or different problems. Similarly, it will be understood that the benefits and advantages described above may relate to one embodiment or may relate to several embodiments.

The claims are not intended to include, and should not be interpreted to include, means-plus- or step-plus-function limitations, unless such a limitation is explicitly recited in a given claim using the phrase(s) "means for" or "step for," respectively.

## Claims

1. A bio-compatible anchor (101) comprising:

   a) a collet (202) having a central longitudinal axis (211), a sidewall (212), a first end (213), a second end (214), and a length (215) between the first end (213) and the second end (214), the sidewall (212) defining a plurality of ridges (216) and valleys (217) extending in a direction that is parallel in at least one plane to the central longitudinal axis (211); and
   b) a retaining collar (201) having a passage configured to receive at least the first end (213) of the collet (202);
   c) where the collet (202) is configured to be inserted into the passage of the retaining collar (201) to compress the sidewall (212) along the length of the collet (202) toward the central longitudinal axis (211) to provide a pressure to a surgical cable (103) which, in use, is disposed in the collet,

the pressure being substantially equal at contact regions disposed at substantially equiangular intervals around the circumference of the surgical cable (103), **characterised in that** the pressure is substantially uniform along a majority of the length of the collet.

2. A bio-compatible anchor (101) as in Claim 1, where the ridges (216) and valleys (217) of the sidewall (212) define a plurality of fingers (305) each having an inner end (306) extending radially inward toward the central longitudinal axis (211), the fingers (305) configured to be compressed inwardly toward the central longitudinal axis (211) to apply pressure to the contact area of a cable (103) disposed in the collet (202).

3. A bio-compatible anchor (101) as in Claim 1 or 2, where the pressure is substantially equal at contact regions disposed at substantially equiangular intervals around the circumference of the surgical cable (103), for example wherein the substantially uniform pressure comprises a pressure of 17.237 Mpa to 24.132 Mpa (2500 to 3500 psi).

4. A bio-compatible anchor (101) as in Claim 1, further comprising a crimping tube (902) coupled to the collar (201) and parallel to the length (207) of the collar (201), for example further comprising:

   d) a length of cable (103) having a portion crimped within the crimping tube (902).

5. A bio-compatible anchor (101) as in claim 1, further comprising a pair of opposing attachment tabs coupled to the collar (201) and extending perpendicular to the length (207) of the collar (201).

6. A bio-compatible anchor (101) as in Claim 3, where the relative distribution of the pressure remains substantially constant over cable (103) diameter shrinkages of up to 15%.

7. A bio-compatible anchor (101) as in Claim 1, where the second end (214) of the collet (202) is larger than the first end (213) of the collet (202) such that an outer profile of the collet (202) is tapered, and where the passage of the collar (201) is tapered to correspond to the taper of the outer profile of the collet (202), for example where the valleys (217) of the collet (202) define an inner profile of the collet (202) that is not tapered.

8. A bio-compatible anchor (101) as in Claim 1, where the collet (202) is configured to be resilient, for example where at least one of the collet (202) and collar (201) comprises polymer.

9. A bio-compatible anchor (101) as in claim 1, where the ridges (216) and valleys (217) of the sidewall (212) define 7-11 radial fingers (305), for example where the sidewall (212) has a thickness of 0.05 - 0.305mm (0.002 - 0.012 inches), for example where at least one of the collar (201) and the collet (202) comprises a metallic alloy, for example where the metallic alloy comprises titanium, for example further comprising a crimping tube (902) coupled to the collar (201) and parallel to the length (207) of the collar (201), for example further comprising:

   d) a length of cable (103) having a portion crimped within the crimping tube (902).

10. A bio-compatible anchor (101) as in Claim 6, further comprising a pair of opposing attachment tabs coupled to the collar (201) and extending perpendicular to the length (207) of the collar (201).

11. A bio-compatible anchor (101) as in claim 1, where the biocompatible anchor is configured to one of:

   (a) provide the substantially uniform pressure to a surgical cable (103) having a relaxed or non-tensioned diameter of between 0.5 mm and 2 mm; and
   (b) substantially resist longitudinal movement of the surgical cable (103) relative to the collet (202) at tensions in the surgical cable (103) of up to 600 Newtons.

12. A bio-compatible anchor (101) as in claim 1, where the collar (201) comprises a first end (205), a second end (206), and a length (207) between the first end (205) and the second end (206), where the passage extends through and between the first end (205) and the second end (206), for example where the collar (201) is configured to receive the first end (213) of the collet (202) through the first end (205) of the collar (201), and is configured to progressively compress the sidewall (212) along the length (215) of the collet (202) as the first end (213) of the collet (202) is pressed into the passage toward the second end (206) of the collar (201), for example where the first end (205) of the collar (201) includes an enlarged flange (208) extending outwardly relative to the passage.

13. A kit comprising the anchor (101) of any preceding claim and an installation tool (700), the tool comprising;

   a) an upper member (701) configured to contact the collet (202) of the bio-compatible anchor;
   b) a lower member (702) configured to contact the collar (201) of the bio-compatible anchor (101) and movable relative to the upper member (701) to insert the collet (202) into the collar (201); for example where the installation tool (700) is configured to grip a surgical cable (103) extending through the bio-compatible anchor (101) when the bio-compatible anchor (101) is in contact with the upper and lower member (702)s of the installation tool (700), for example further comprising

c) a lever (705) including a passageway (706) configured to receive the surgical cable (103); and

d) a tension member (707) configured to resist withdrawal of the surgical cable (103) from the passageway (706) to maintain a tension in the surgical cable (103), for example where the upper member (701) is pivotally coupled to the lower member (702), for example where one of:

> (i) the upper member (701) is coupled to the lower member (702) in a scissor-like configuration; and
> (ii) the lower member (702) comprises a forked portion (703) configured to receive the collar (201), the upper member (701) comprises a forked portion (704) configured to receive the surgical cable (103).

14. A kit comprising a bio-compatible anchor (101) as in any of Claims 1 to 12 and one of:

> i) a package within which the bio-compatible anchor (101)is disposed, where the bio-compatible anchor (101)is sterile, and
> ii) a washer (104) having an inner transverse dimension that is greater than an outer transverse dimension of a portion of the collar (201) between the first end (205) and the second end (206) of the collar (201), and smaller than an outer transverse dimension of the flange (208).

**Patentansprüche**

1. Biokompatibler Anker (101), der Folgendes umfasst:

> a) eine Klemmhülse (202) mit einer zentralen Längsachse (211), einer Seitenwand (212), einem ersten Ende (213), einem zweiten Ende (214) und einer Länge (215) zwischen dem ersten Ende (213) und dem zweiten Ende (214), wobei die Seitenwand (212) mehrere Höhen (216) und Tiefen (217) definiert, die in einer Richtung verlaufen, die in wenigstens einer Ebene parallel zur zentralen Längsachse (211) ist; und
> b) einen Haltekragen (201) mit einem Kanal, der zum Aufnehmen wenigstens des ersten Endes (213) der Klemmhülse (202) konfiguriert ist;

c) wobei die Klemmhülse (202) zum Einführen in den Kanal des Haltekragens (201) konfiguriert ist, um die Seitenwand (212) entlang der Länge der Klemmhülse (202) in Richtung der zentralen Längsachse (211) zu komprimieren, um einen Druck auf ein chirurgisches Kabel (103) aufzubringen, das beim Gebrauch in der Klemmhülse angeordnet ist, wobei der Druck im Wesentlichen gleich in Kontaktregionen ist, die in im Wesentlichen gleichwinkligen Intervallen um den Umfang des chirurgischen Kabels (103) angeordnet sind,

**dadurch gekennzeichnet, dass** der Druck im Wesentlichen gleichmäßig über eine Mehrheit der Länge der Klemmhülse verteilt ist.

2. Biokompatibler Anker (101) nach Anspruch 1, wobei die Höhen (216) und Tiefen (217) der Seitenwand (212) mehrere Finger (305) jeweils mit einem inneren Ende (306) definieren, das radial einwärts in Richtung der zentralen Längsachse (211) verläuft, wobei die Finger (305) zum Komprimieren einwärts in Richtung der zentralen Längsachse (211) konfiguriert sind, um Druck auf den Kontaktbereich eines in der Klemmhülse (202) angeordneten Kabels (103) aufzubringen.

3. Biokompatibler Anker (101) nach Anspruch 1 oder 2, wobei der Druck im Wesentlichen gleich in Kontaktregionen ist, die in im Wesentlichen gleichwinkligen Intervallen um den Umfang des chirurgischen Kabels (103) angeordnet sind, wobei zum Beispiel der im Wesentlichen gleichförmige Druck einen Druck von 17,237 MPa bis 24,132 MPa (2500 bis 3500 psi) umfasst.

4. Biokompatibler Anker (101) nach Anspruch 1, der ferner eine Crimpröhre (902) umfasst, die mit dem Kragen (201) gekoppelt und parallel zur Länge (207) des Kragens (201) ist, zum Beispiel ferner Folgendes umfassend:

> d) eine Länge Kabel (103) mit einem Abschnitt, der in der Crimpröhre (902) gecrimpt ist.

5. Biokompatibler Anker (101) nach Anspruch 1, der ferner ein Paar gegenüberliegende Befestigungszungen umfasst, die mit dem Kragen (201) gekoppelt sind und lotrecht zur Länge (207) des Kragens (201) verlaufen.

6. Biokompatibler Anker (101) nach Anspruch 3, wobei die relative Verteilung des Drucks im Wesentlichen konstant über Schrumpfungen des Durchmessers des Kabels (103) von bis zu 15 % bleibt.

**7.** Biokompatibler Anker (101) nach Anspruch 1, wobei das zweite Ende (214) der Klemmhülse (202) größer ist als das erste Ende (213) der Klemmhülse (202), so dass ein Außenprofil der Klemmhülse (202) konisch zuläuft, und wobei der Kanal des Kragens (201) konisch zuläuft, so dass er der Konizität des Außenprofils der Klemmhülse (202) entspricht, wobei zum Beispiel die Tiefen (217) der Klemmhülse (202) ein Innenprofil der Klemmhülse (202) definieren, das nicht konisch zuläuft.

**8.** Biokompatibler Anker (101) nach Anspruch 1, wobei die Klemmhülse (202) so konfiguriert ist, dass sie elastisch ist, wobei zum Beispiel wenigstens eines aus Klemmhülse (202) und Kragen (201) Polymer umfasst.

**9.** Biokompatibler Anker (101) nach Anspruch 1, wobei die Höhen (216) und Tiefen (217) der Seitenwand (212) 7-11 radiale Finger (305) definieren, wobei zum Beispiel die Seitenwand (212) eine Dicke von 0,05 - 0,305 mm (0,002 - 0,012 Zoll) hat, wobei zum Beispiel wenigstens eines aus Kragen (201) und Klemmhülse (202) eine metallische Legierung umfasst, wobei zum Beispiel die metallische Legierung Titan umfasst, zum Beispiel ferner umfassend eine Crimpröhre (902), die mit dem Kragen (201) gekoppelt und parallel zur Länge (207) des Kragens (201) ist, zum Beispiel ferner Folgendes umfassend:

d) eine Länge Kabel (103) mit einem Abschnitt, der innerhalb der Crimpröhre (902) gecrimpt ist.

**10.** Biokompatibler Anker (101) nach Anspruch 6, der ferner ein Paar gegenüberliegende Befestigungszungen umfasst, die mit dem Kragen (201) gekoppelt sind und lotrecht zur Länge (207) des Kragens (201) verlaufen.

**11.** Biokompatibler Anker (101) nach Anspruch 1, wobei der biokompatible Anker für eines der Folgenden konfiguriert ist:

(a) Bereitstellen des im Wesentlichen gleichmäßigen Drucks auf ein chirurgisches Kabel (103) mit einem entspannten oder nicht gespannten Durchmesser zwischen 0,5 mm und 2 mm; und (b) im Wesentlichen Widerstehen einer Längsbewegung des chirurgischen Kabels (103) relativ zur Klemmhülse (202) bei Spannungen im chirurgischen Kabel (103) von bis zu 600 Newton.

**12.** Biokompatibler Anker (101) nach Anspruch 1, wobei der Kragen (201) Folgendes aufweist: ein erstes Ende (205), ein zweites Ende (206) und eine Länge (207) zwischen dem ersten Ende (205) und dem zweiten Ende (206), wobei der Kanal durch und zwischen dem ersten Ende (205) und dem zweiten Ende (206) verläuft, wobei zum Beispiel der Kragen (201) zum Aufnehmen des ersten Endes (213) der Klemmhülse (202) durch das erste Ende (205) des Kragens (201) konfiguriert ist und zum progressiven Komprimieren der Seitenwand (212) entlang der Länge (215) der Klemmhülse (202) konfiguriert ist, während das erste Ende (213) der Klemmhülse (202) in den Kanal in Richtung des zweiten Endes (206) des Kragens (201) gepresst wird, wobei zum Beispiel das erste Ende (205) des Kragens (201) einen vergrößerten Flansch (208) aufweist, der auswärts relativ zu dem Kanal verläuft.

**13.** Kit, das den Anker (101) nach einem vorherigen Anspruch und ein Installationswerkzeug (700) umfasst, wobei das Werkzeug Folgendes umfasst:

a) ein oberes Element (701), konfiguriert zum Kontaktieren der Klemmhülse (202) des biokompatiblen Ankers; b) ein unteres Element (702), das zum Kontaktieren des Kragens (201) des biokompatiblen Ankers (101) konfiguriert und relativ zum oberen Element (701) zum Einführen der Klemmhülse (202) in den Kragen (201) beweglich ist, wobei zum Beispiel das Installationswerkzeug (700) so konfiguriert ist, dass es ein chirurgisches Kabel (103) ergreift, das durch den biokompatiblen Anker (101) verläuft, wenn der biokompatible Anker (101) mit dem oberen und unteren Element (702) des Installationswerkzeugs (700) in Kontakt ist, zum Beispiel ferner Folgendes umfassend: c) einen Hebel (705) mit einem Durchgang (706), der zum Aufnehmen des chirurgischen Kabels (103) konfiguriert ist; und d) ein Spannelement (707), das so konfiguriert ist, dass es einem Herausziehen des chirurgischen Kabels (103) aus dem Durchgang (706) widersteht, um eine Spannung in dem chirurgischen Kabel (103) zu halten, wobei zum Beispiel das obere Element (701) schwenkbar mit dem unteren Element (702) gekoppelt ist, wobei zum Beispiel eines der Folgenden gilt:

(i) das obere Element (701) ist mit dem unteren Element (702) in einer scherenähnlichen Konfiguration gekoppelt; und (ii) das untere Element (702) umfasst einen gegabelten Abschnitt (703), der zum Aufnehmen des Kragens (201) konfiguriert ist, das obere Element (701) umfasst einen gegabelten Abschnitt (704), der zum Aufnehmen des chirurgischen Kabels (103) konfiguriert ist.

**14.** Kit, das einen biokompatiblen Anker (101) nach einem der Ansprüche 1 bis 12 und eines der Folgenden umfasst:

i) ein Paket, in dem der biokompatible Anker (101) angeordnet ist, wobei der biokompatible Anker (101) steril ist, und

ii) eine Zwischenscheibe (104) mit einer inneren Querabmessung, die größer als eine äußere Querabmessung eines Abschnitts des Kragens (201) zwischen dem ersten Ende (205) und dem zweiten Ende (206) des Kragens (201) und kleiner als eine äußere Querabmessung des Flansches (208) ist.

## Revendications

1. Ancre biocompatible (101) comprenant :

a) une pince de serrage (202) ayant un axe central longitudinal (211), une paroi latérale (212), une première extrémité (213), une deuxième extrémité (214), et une longueur (215) entre la première extrémité (213) et la deuxième extrémité (214), la paroi latérale (212) définissant une pluralité de crêtes (216) et de creux (217) s'étendant dans une direction qui est parallèle dans au moins un plan à l'axe central longitudinal (211) ; et

b) un collier de retenue (201) ayant un passage configuré pour recevoir au moins la première extrémité (213) de la pince de serrage (202) ;

c) où la pince de serrage (202) est configurée pour être insérée jusque dans le passage du collier de retenue (201) pour comprimer la paroi latérale (212) le long de la longueur de la pince de serrage (202) vers l'axe central longitudinal (211) pour fournir une pression sur un câble chirurgical (103) qui, pendant l'emploi, est disposé dans la pince de serrage, la pression étant substantiellement égale au niveau de régions de contact disposées à intervalles substantiellement équiangulaires autour de la circonférence du câble chirurgical (103),

**caractérisée en ce que** la pression est substantiellement uniforme le long d'une majorité de la longueur de la pince de serrage.

2. Ancre biocompatible (101) selon la revendication 1, où les crêtes (216) et les creux (217) de la paroi latérale (212) définissent une pluralité de doigts (305) ayant chacun une extrémité interne (306) s'étendant de manière radiale vers l'axe central longitudinal (211), les doigts (305) configurés pour être comprimés vers l'intérieur vers l'axe central longitudinal (211) pour appliquer de la pression sur la surface de contact d'un câble (103) disposé dans la pince de serrage (202).

3. Ancre biocompatible (101) selon la revendication 1 ou 2, où la pression est substantiellement égale au niveau de régions de contact disposées à intervalles substantiellement équiangulaires autour de la circonférence du câble chirurgical (103), par exemple dans laquelle la pression substantiellement uniforme comprend une pression de 17,237 Mpa à 24,132 Mpa (2 500 à 3 500 psi).

4. Ancre biocompatible (101) selon la revendication 1, comprenant en outre un tube de sertissage (902) couplé au collier (201) et parallèle à la longueur (207) du collier (201), par exemple comprenant en outre :

d) une longueur de câble (103) ayant une partie sertie au sein du tube de sertissage (902).

5. Ancre biocompatible (101) selon la revendication 1, comprenant en outre une paire de pattes de fixation opposées couplées au collier (201) et s'étendant perpendiculaires à la longueur (207) du collier (201).

6. Ancre biocompatible (101) selon la revendication 3, où la répartition relative de la pression reste substantiellement constante sur les rétrécissements du diamètre du câble (103) d'un maximum de 15 %.

7. Ancre biocompatible (101) selon la revendication 1, où la deuxième extrémité (214) de la pince de serrage (202) est plus large que la première extrémité (213) de la pince de serrage (202) de sorte qu'un profil externe de la pince de serrage (202) est effilé, et où le passage du collier (201) est effilé pour correspondre à l'effilement du profil externe de la pince de serrage (202), par exemple où les creux (217) de la pince de serrage (202) définissent un profil interne de la pince de serrage (202) qui n'est pas effilé.

8. Ancre biocompatible (101) selon la revendication 1, où la pince de serrage (202) est configurée pour être résiliente, par exemple où au moins un élément parmi la pince de serrage (202) et le collier (201) comprend un polymère.

9. Ancre biocompatible (101) selon la revendication 1 où les crêtes (216) et les creux (217) de la paroi latérale (212) définissent de 7 à 11 doigts radiaux (305), par exemple où la paroi latérale (212) a une épaisseur de 0,05 à 0,305 mm (0,002 à 0,012 pouces), par exemple où au moins un élément parmi le collier (201) et la pince de serrage (202) comprend un alliage métallique, par exemple où l'alliage métallique comprend du titane, par exemple comprenant en outre un tube de sertissage (902) couplé au collier (201) et parallèle à la longueur (207) du collier (201), par exemple comprenant en outre :

d) une longueur de câble (103) ayant une partie sertie au sein du tube de sertissage (902).

10. Ancre biocompatible (101) selon la revendication 6, comprenant en outre une paire de pattes de fixation opposées couplées au collier (201) et s'étendant perpendiculaires à la longueur (207) du collier (201).

11. Ancre biocompatible (101) selon la revendication 1, où l'ancre biocompatible est configurée pour :

a) soit fournir la pression substantiellement uniforme sur un câble chirurgical (103) ayant un diamètre détendu ou sans tension situé entre 0,5 mm et 2 mm ; et
b) soit résister substantiellement au mouvement longitudinal du câble chirurgical (103) par rapport à la pince de serrage (202) à des tensions dans le câble chirurgical (103) s'élevant jusqu'à 600 newtons.

12. Ancre biocompatible (101) selon la revendication 1, où le collier (201) comprend une première extrémité (205), une deuxième extrémité (206), et une longueur (207) entre la première extrémité (205) et la deuxième extrémité (206), où le passage s'étend à travers et entre la première extrémité (205) et la deuxième extrémité (206), par exemple où le collier (201) est configuré pour recevoir la première extrémité (213) de la pince de serrage (202) à travers la première extrémité (205) du collier (201), et est configuré pour comprimer progressivement la paroi latérale (212) le long de la longueur (215) de la pince de serrage (202) alors que la première extrémité (213) de la pince de serrage (202) est appuyée jusque dans le passage vers la deuxième extrémité (206) du collier (201), par exemple où la première extrémité (205) du collier (201) inclut une bride élargie (208) s'étendant vers l'extérieur par rapport au passage.

13. Trousse comprenant l'ancre (101) de l'une quelconque des revendications précédentes et un outil d'installation (700), l'outil comprenant ;

a) un élément supérieur (701) configuré pour entrer en contact avec la pince de serrage (202) de l'ancre biocompatible ;
b) un élément inférieur (702) configuré pour entrer en contact avec le collier (201) de l'ancre biocompatible (101) et mobile par rapport à l'élément supérieur (701) pour insérer la pince de serrage (202) jusque dans le collier (201) ; par exemple où l'outil d'installation (700) est configuré pour saisir un câble chirurgical (103) s'étendant à travers l'ancre biocompatible (101) quand l'ancre biocompatible (101) est en contact avec les éléments supérieur et inférieur (702) de l'outil d'installation (700), par exemple comprenant en outre
c) un levier (705) incluant une voie de passage (706), configuré pour recevoir le câble chirurgical (103) ; et
d) un élément de tension (707) configuré pour résister au retrait du câble chirurgical (103) de la voie de passage (706) pour maintenir une tension dans le câble chirurgical (103), par exemple où l'élément supérieur (701) est couplé en rotation à l'élément inférieur (702), par exemple où :

i) soit l'élément supérieur (701) est couplé à l'élément inférieur (702) dans une configuration semblable à des ciseaux ; et
ii) soit l'élément inférieur (702) comprend une partie fourchue (703) configurée pour recevoir le collier (201), l'élément supérieur (701) comprend une partie fourchue (704) configurée pour recevoir le câble chirurgical (103).

14. Trousse comprenant une ancre biocompatible (101) selon l'une quelconque des revendications 1 à 12 et :

i) soit un emballage à l'intérieur duquel l'ancre biocompatible (101) est disposée, où l'ancre biocompatible (101) est stérile, et
ii) soit une rondelle (104) ayant une dimension transversale interne qui est supérieure à une dimension transversale externe d'une partie du collier (201) entre la première extrémité (205) et la deuxième extrémité (206) du collier (201), et inférieure à une dimension transversale externe de la bride (208).

FIG. 1A

FIG. 1B

FIG. 2

FIG. 3A

FIG. 3B

FIG. 3C

FIG. 3D

FIG. 3E

EP 2 852 331 B1

19

EP 2 852 331 B1

FIG. 4A

FIG. 4B

FIG. 4C

FIG. 4D

20

EP 2 852 331 B1

*FIG. 5A*

*FIG. 5B*

PRIOR ART

FIG. 6

EP 2 852 331 B1

**FIG. 7**

FIG. 8

*FIG. 9*

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7625373 B
- US 6589246 B
- US 5258015 A
- US 2008004624 A1